# EUROPÄISCHE PATENTSCHRIFT

(11) **EP 2 274 291 B1**
(45) Veröffentlichungstag und Bekanntmachung des Hinweises auf die Patenterteilung: **01.06.2016**
(21) Anmeldenummer: 09737784.0
(22) Anmeldetag: 31.03.2009
(51) Int. Cl.: C07D 263/04, C07D 295/088, C07D 295/037, C07D 295/145, C07D 295/108, C07C 211/63, C07C 219/06, C07C 255/24, C07F 9/54, C07F 9/6568, H01B 1/12, H01M 10/052, H01M 10/0525, H01G 11/06, H01G 11/58

(54) **REAKTIVE IONISCHE FLÜSSIGKEITEN**
REACTIVE IONIC LIQUIDS
LIQUIDES IONIQUES RÉACTIFS

(30) Priorität: 29.04.2008 DE 102008021271
(43) Veröffentlichungstag der Anmeldung: 19.01.2011
(60) Teilanmeldung: 16161031.6
(73) Patentinhaber: BASF SE, 67056 Ludwigshafen am Rhein (DE)
(72) Erfinder: SCHMIDT, Michael, 64342 Seeheim-Jugenheim (DE); IGNATYEV, Nikolai (Mykola), 47058 Duisburg (DE); PITNER, William-Robert, 60599 Frankfurt (DE)
(74) Vertreter: BASF IP Association
(86) Internationale Anmeldenummer: PCT/EP2009/002329
(87) Internationale Veröffentlichungsnummer: WO 2009/132740

(56) Entgegenhaltungen:
- WO-A1-01/25326
- WO-A1-2004/035542
- WO-A2-2007/147222
- CN-A- 101 085 762
- CN-A- 101 210 000
- DE-A1- 2 609 474
- JP-A- 2006 206 517
- JP-A- 2007 161 678
- US-A- 3 723 512
- US-A1- 2006 210 876
- US-A1- 2009 036 715
- LEE J S ET AL: "Ionic liquids containing an ester group as potential electrolytes" ELECTROCHEMISTRY COMMUNICATION, ELSEVIER, AMSTERDAM, NL, Bd. 8, Nr. 3, 1. März 2006 (2006-03-01), Seiten 460-464, XP025182092 ISSN: 1388-2481 [gefunden am 2006-03-01]
- EGASHIRA M ET AL: "The preparation of quaternary ammonium-based ionic liquid containing a cyano group and its properties in a lithium battery electrolyte" JOURNAL OF POWER SOURCES, ELSEVIER, AMSTERDAM, NL, Bd. 138, Nr. 1-2, 15. November 2004 (2004-11-15), Seiten 240-244, XP004618359 ISSN: 0378-7753
- EGASHIRA M ET AL: "Charge-discharge and high temperature reaction of LiCoO2 in ionic liquid electrolytes based on cyano-substituted quaternary ammonium cation" JOURNAL OF POWER SOURCES, ELSEVIER, AMSTERDAM, NL, Bd. 160, Nr. 2, 6. Oktober 2006 (2006-10-06), Seiten 1387-1390, XP025084432 ISSN: 0378-7753 [gefunden am 2006-10-06]
- EGASHIRA M ET AL: "Cyano-containing quaternary ammonium-based ionic liquid as a 'co-solvent' for lithium battery electrolyte" JOURNAL OF POWER SOURCES, ELSEVIER, AMSTERDAM, NL, Bd. 146, Nr. 1-2, 26. August 2005 (2005-08-26), Seiten 685-688, XP025269111 ISSN: 0378-7753 [gefunden am 2005-08-26]
- DATABASE CAPLUS [Online] CHEMICAL ABSTRACTS SERVICE, COLUMBUS, OHIO, US; 20. Mai 2000 (2000-05-20), HAYASHI, KATSUYA: "Electrolyte and secondary lithium battery which uses the electrolyte" XP002531096 gefunden im STN Database accession no. 2005:489759 -& JP 2005 149982 A (NIPPON TELEGRAPH AND TELEPHONE CORP., JAPAN) 9. Juni 2005 (2005-06-09)
- OKOTURO ET AL: "Temperature dependence of viscosity for room temperature ionic liquids" JOURNAL OF ELECTROANALYTICAL CHEMISTRY AND INTERFACIALELECTROCHEMISTRY, ELSEVIER, AMSTERDAM, NL, Bd. 568, 1. Januar 2004 (2004-01-01), Seiten 167-181, XP002334479 ISSN: 0022-0728
- HAJIME MATSUMOTO ET AL: "Room temperature ionic liquids based on small aliphatic ammonium cations and asymmetric amide anions" CHEMICAL COMMUNICATIONS, CHEMICAL SOCIETY, LONDON, GB, 1. Januar 2002 (2002-01-01), Seiten 1726-1727, XP002312379 ISSN: 0009-241X
- ZHANG Q ET AL: "Physicochemical properties of nitrile-functionalized ionic liquids" JOURNAL OF PHYSICAL CHEMISTRY B 20070322 AMERICAN CHEMICAL SOCIETY US, Bd. 111, Nr. 11, 22. März 2007 (2007-03-22), Seiten 2864-2872, XP002531115
- DINH Q N ET AL: "Synthesis and characterization of quaternary ammonium-based ionic liquids containing an alkyl carbonate group" BULLETIN OF THE KOREAN CHEMICAL SOCIETY, KOREAN CHEMICAL SOCIETY, SEOUL, KR, Bd. 28, Nr. 12, 1. Dezember 2007 (2007-12-01), Seiten 2299-2302, XP009117677 ISSN: 0253-2964
- YIM, TAEEUN ET AL: "Synthesis and properties of pyrrolidinium and piperidinium bis(trifluoromethanesulfonyl)imide ionic liquids with allyl substituents" BULLETIN OF THE KOREAN CHEMICAL SOCIETY , 28(9), 1567-1572 CODEN: BKCSDE; ISSN: 0253-2964, 2007, XP002558013
- ZHANG, QING-SHAN ET AL: "Synthesis of ionic liquids based on the N-methyl-N-allylmorpholinium cation" GAODENG XUEXIAO HUAXUE XUEBAO , 26(2), 340-342 CODEN: KTHPDM; ISSN: 0251-0790, 2005, XP009126608
- MA K ET AL: "Design criteria for ionic liquid crystalline phases of phosphonium salts with three equivalent long n-alkyl chains" JOURNAL OF ORGANIC CHEMISTRY 20090306 AMERICAN CHEMICAL SOCIETY US, Bd. 74, Nr. 5, 6. März 2009 (2009-03-06), Seiten 2088-2098, XP002558089
- YOUJI HUAXUE / CHINESE JOURNAL OF ORGANIC CHEMISTRY., Bd. 27, Nr. 9, 2007, Seiten 1167-1170, XP009126609 SCIENCE PRESS, BEIJING. ISSN: 0253-2786
- GALINSKI M ET AL: "Ionic liquids as electrolytes" ELECTROCHIMICA ACTA 20060815 ELSEVIER LTD GB, Bd. 51, Nr. 26, 15. August 2006 (2006-08-15), Seiten 5567-5580, XP002531097

## Beschreibung

Die Erfindung betrifft reaktive ionische Flüssigkeiten enthaltend organische Kationen mit elektrochemisch reduktionsanfälligen Gruppen bzw. Substituenten sowie Anionen aus Fluoralkylphosphaten, Fluoralkylphosphinaten, Fluoralkylphosphonaten, Acetaten, Triflaten, Methiden, Boraten, Phosphaten und Aluminaten zur Anwendung in elektrochemischen Zellen wie Lithiumionen-Batterien und Doppelschichtkondensatoren.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf.

Unter "reaktiven ionischen Flüssigkeiten" versteht man ionische Flüssigkeiten, die am organischen Kation elektrochemisch reduktionsanfällige Gruppen bzw. Substituenten wie Cyanogruppen, Estergruppen, Carbonatgruppen oder Seitenketten mit Doppelbindungen aufweisen.

In den letzten Jahren haben ionische Flüssigkeiten mehr und mehr Interesse erlangt und eine Vielzahl von Review-Artikeln haben einzigartige Eigenschaften von Ionischen Fluiden (Ionische Flüssigkeiten, Ionic Liquids bzw. abgekürzt "ILs") beschreiben und verschiedene Einsatzpotentiale aufgezeigt.
Insbesondere erscheinen Ionische Flüssigkeiten viel versprechend für den Einsatz in Energiespeichermedien wie Doppelschichtkondensatoren und Batterien mit einem besonderen Fokus auf Elektro- und Hybridfahrzeugen. Herausragende Eigenschaften von Ionischen Flüssigkeiten beinhalten:
- Eine praktische nicht vorhandene Flüchtigkeit und damit sehr hohe Flammpunkte
- Ein sehr großer Flüssigkeitsbereich über teilweise mehrere 100K
- Eine sehr hohe Polarität und damit in der Regel sehr gute Löslichkeit für anorganische und organische Salze

Während Ionische Flüssigkeiten wie zum Beispiel Ethyl-Methyl-Imidazolium Tetrafluoroborat (EMIBF₄) bereits kommerziell in Doppelschichtkondensatoren (Super- oder Ultracaps) eingesetzt werden, gestaltet sich die Verwendung in Batterien insbesondere Lithium-Ionen-Batterien weiterhin schwierig.

Für Batterieanwendungen wurden folgende ionische Flüssigkeiten beinhaltende, Systeme charakterisiert:
- Ionische Flüssigkeiten in Kombination mit einen Li-Salz enthaltenden Elektrolyten
- Ionische Flüssigkeiten in Kombination mit einen Li-Salz enthaltenden Elektrolyten plus Additivzusätze

Hierbei wurden folgende Ionische Flüssigkeiten verwendet:
- Ionische Flüssigkeiten mit AlCl₄ als Anion (*Generation 0*, sehr frühe Arbeiten)
- Ionische Flüssigkeiten mit Imidazolium basierten Kationen und (per)fluorierten anorganischen oder organischen Anionen (*Generation 1*)
- Ionische Flüssigkeiten mit "Nicht-Imidazolium" basierten Kationen und (per)fluorierten anorganischen oder organischen Anionen (*Generation* 2)

AlCl4- basierte Ionische Flüssigkeiten sind extrem hydrolyseempfindlich und reagieren mit Wasser unter Freisetzung von Salzsäure HCl. Die Entwicklung für Batteriesysteme wurde aufgrund dessen eingestellt. Imidazolium basierte ILs zeigen eine unzureichende reduktive Stabilität und werden daher für die kommerzielle Anwendung in Hochenergiebatterien als nicht viel versprechend angesehen. Ionische Flüssigkeiten mit anorganischen Anionen, insbesondere mit BF4- verursachen, insbesondere in den ersten Lade/Entladezyklen, einen deutlichen Kapazitätsabfall. Die zurzeit am besten für Li-Ionen Batterien geeigneten Ionischen Flüssigkeiten verwenden N,N Di-Alkyl-pyrrollidinium in Kombination mit Bis(trifluormethyl)imiden (siehe z.B. JP 2006-260952).
Allerdings verursachen selbst diese Ionischen Flüssigkeiten noch immer einen signifikanten Rückgang der Leistungsdichten von Lithium-lonen Batterien.
Grund hierfür ist insbesondere die hohe Viskosität elektrochemisch stabiler Ionischer Flüssigkeiten. Dies führt zu einer deutlich geringeren LithiumIonen-Leitfähigkeit des IL basierten Elektrolyten im Vergleich zu Standard Elektrolytsystemen ohne Ionische Flüssigkeiten (O. Borodin et al., J. of Physical Chemistry B, 2006, 110 (34), p. 16879-16886). Demzufolge zeigen Lithium-Ionen-Batterien mit IL basierten Elektrolyten noch heute eine deutlich geringe Leistungsdichte und Belastbarkeit im Vergleich zu LithiumIonen-Batterien mit Standardelektrolyten dementsprechender.
Gerade letzteres ist für Anwendungen in Elektro- und Hybridfahrzeugen als sehr kritisch anzusehen und verhindert - trotz einem Gewinn an Sicherheit - den Einsatz IL basierter Elektrolyte.
Lee et al. (Electrochem. Comm. 8 (2006) 460 konnten zeigen, dass die Verwendung von Imidazolium basierten ILs mit Esterliganden am Stickstoff in Batterieelektrolyten zu einer Verbesserung der Lithiumleitfähigkeit und des Diffusionskoeffizienten von Li-Ionen führt.
Allerdings sind diese Imidazolium-basierten Ionischen Fluide elektrochemisch nicht stabil genug.
M. Egashira et al. (Journal of Power Sources 138 (2004), Seiten 240 to 244 und Journal of Power Sources 146 (2005), Seiten 685 to 688) befassen sich mit ionischen Flüssigkeiten auf Basis von cyanosubstituierten quartemären Imidazoliumsalzen als Lösungsmittel in Elektrolytmischungen für Lithium-Ionen-Batterien.

US 2006/210876 A1 beschreibt eine elektrochemische Zelle umfassend eine Elektrolytmischung mit ionischen Flüssigkeiten als Lösungsmittel. Die Elektrolytmischung enthält dabei zwischen 1 und 50 % einer ionischen Flüssigkeit mit einem Ammoniumkation, das mit einer carbonathaltigen Gruppe substituiert ist.

Aufgabe der vorliegenden Erfindung war es daher, ionische Flüssigkeiten zu entwickeln, die eine hohe thermische Stabilität, sehr gute Oxidationsstabilität und geringe Korrosivität sowie kostengünstig synthetisierte Anionen aufweisen und die o.g. Nachteile nicht aufweisen.

Die vorliegende Aufgabe wird gelöst durch ionische Flüssigkeiten der allgemeinen Formel I

K⁺ A⁻ (I)

worin bedeuten:
K⁺ ein Kation, vorzugsweise reduktionsstabil, ausgewählt aus der Gruppe der allgemeinen Formeln 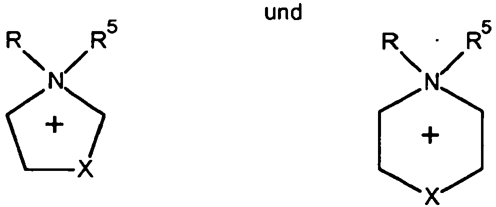 wobei
   X CH₂, O, S oder NR'
   R' -(CH₂)ₙ-CN, Alkyl C₁ bis C₁₆, vorzugsweise Methyl, Ethyl, Propyl, H
   R H, Alkyl C₁ bis C₁₆, vorzugsweise, Methyl, Ethyl, Propyl
   R5 -(CH₂)ₙ-O-C(O)-R, -(CH₂)ₙ-C(O)-OR oder -(CH₂)ₙ-HC=CH-R, wobei einzelne der CH₂-Gruppen durch S oder NR ersetzt sein können mit n = 1 bis 8
   und
A⁻ ein Anion ausgewählt aus der Gruppe 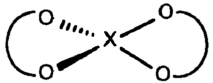 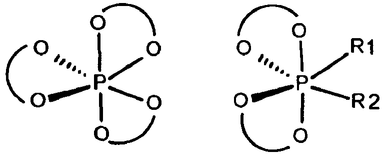 wobei 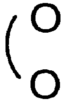
   ein 1,2- oder 1,3- Diol, eine 1,2 -oder 1,3- Dicarbonsäure oder 1,2- oder 1,3- Hydroxycarbonsäure
X B
R₁ bis R₂ Halogen, insbesondere F, und/oder ein fluorierter oder nicht fluorierter Alkoxy- oder Carboxy-Rest
ist.

Gegenüber herkömmlichen ionischen Flüssigkeiten zeichnen sich die erfindungsgemäßen reaktiven ionischen Flüssigkeiten dadurch aus, dass sie am organischen Kation elektrochemisch reduktionsanfällige Gruppen/Substituenten bzw. Seitenketten aufweisen. Dies sind insbesondere
• Estergruppen -R-C(O)-OR oder -R-O-C(O)-R
in Doppelbindungen in den Seitenketten -R=CH=CH-R

Überraschenderweise bilden die erfindungsgemäßen reaktiven ionischen Flüssigkeiten bei Potentialen zwischen ca. 2 V und 0.9V gegen Li/Li⁺ deutlich eher eine passivierende Deckschicht als Ethylencarbonat (0.7 bis 0.8V gegen Li/Li⁺). Diese Deckschicht ist elektronisch passivierend, aber für Lithium-Ionen durchlässig. Ein weiterer Vorteil besteht in der ausgezeichneten Oxidationsstabilität mit > 5 V gegen Li/Li⁺. Anders als viele andere zur Zeit eingesetzte teilweise hoch toxische (z.B. Propansulton) Additive sind die erfindungsgemäßen reaktiven ionischen Flüssigkeiten nicht flüchtig und besitzen keinen messbaren Dampfdruck.

Vorzugsweise handelt es sich bei den Kationen K⁺ der erfindungsgemäßer ionischen Flüssigkeiten um Kationen ausgewählt aus der allgemeinen Formel 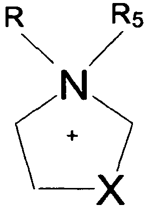 wobei
- X: CH₂, O, S oder NR'
- R': -(CH₂)ₙ-CN, Methyl, Ethyl, Propyl, Butyl, H
- R: H, Methyl, Ethyl, Propyl, Butyl
- R₅: -(CH₂)ₙ-O-C(O)-R, -(CH₂)ₙ-C(O)-OR, oder -(CH₂)ₙ-HC=CH-R, wobei einzelne der CH₂-Gruppen durch S oder NR ersetzt sein können mit n = 1 bis 8

Bei den Anionen A- der erfindungsgemäßen ionischen Flüssigkeiten handelt es sich um folgende Anionen: spiro-Oxo-Borate und spiro-Oxo-Phosphate, insbesondere bevorzugt sind spiro-Oxo-Borate.

Ein weiterer Gegenstand ist ein Elektrolyt enthaltend mindestens ein Leitsalz, ein aprotisches Lösungsmittel oder Lösungsmittelgemisch, mindestens eine erfindungsgemäße ionische Flüssigkeit der oben genannten Formel I sowie gegebenenfalls weitere Additive.

In einer bevorzugten Ausführungsform (z.B. bei Verwendung des Elektrolyten in Lithium- oder Lithiumionen-Batterien) ist das Leitsalz ein Lithiumleitsalz wie LiPF₆, LiN(SO₂CF₃)₂, LiN(SO₂C₂F₅)₂, LiF₃P(C₂F₅)₃, LiF₃P(C₄F₉)₃, LiB(C₂O₄)₂ oder LiF₂B(C₂O₄)₂.

In einer weiteren bevorzugten Ausführungsform (z.B. bei Verwendung des Elektrolyten in Doppelschicht- oder Superkondensatoren) ist das Leitsalz eine Verbindung aus der Gruppe N(C₂H₅)₄BF₄ N(C₂H₅)₄PF₆, N(C₂H₅)₃(CH₃) BF₄, N(C₂H₅)₃(CH₃) PF₆, N(C₂H₅)₄N(SO₂CF₃)₂, N(C₂H₅)₃(CH₃) N(SO₂CF₃)₂, N(C₂H₅)₄F₃P(C₂F₅)₃, N(C₂H₅)₃(CH₃)F₃P(C₂F₅)₃

Vorzugsweise besteht das aprotische Lösungsmittel des Elektrolyten aus organischen offenkettigen oder zyklischen Carbonaten, Carbonsäureestern, Nitrilen, Ethern oder Lactonen oder einem Gemisch daraus. Nitrile, insbesondere Acetonitril, werden als Lösungsmittel vorzugsweise in Doppelschichtkondensatoren eingesetzt.

Ein weiterer Gegenstand der vorliegenden Erfindung ist ein Verfahren zur Herstellung von ionischen Flüssigkeiten der Formel I
- Herstellen von heterozyklischen Kationen K⁺ mit Alkenyl- oder Carboxylat-haltigen Seitenketten gemäß Anspruch 1 als Oniumchloride oder -bromide aus den entsprechenden Aminen, Halogencarboxylaten, oder Alkenylhalogeniden
   nach herkömmlichen nasschemischen Methoden.
- Umsetzung dieser kationischen Oniumchloride oder -bromide mit den entsprechenden anionischen
   Lithium- oder Kalium-Boraten oder -Phosphaten im wässrigen und/oder alkoholischen Milieu oder organisches Lösungsmittels oder ohne Lösungsmittel.

Die Herstellung der Kationen sind dem Fachmann bekannt und können nach Verfahren wie sie z.B. in P. Wasserscheid und T. Welton (Eds.) "Ionic Liquids in Synthesis", Wiley -VCH, 2003, S. 7-40 allgemein oder für Imidazolium-Kationen in N. Gathergood, P.J. Scammells, Aust. J. Chem, 55 (2002), Nr. 9, S. 557-560; E. Alcalde, M. Gisbert, L. Perez-Garcia, Heterocycles, 43 (1996(, Nr.3, S. 567-580; Z. Fei, D. Zhao, T. J. Geldbach, R. Scopelliti, P.J. Dyson, Chem. Europ. J., 10 (2004), Nr. 19, S. 4886-4893; D. Liu, Ji. Gui, X. Zhu, L. Song, Z. Sun, Synth. Commun. , 37 (2007), Nr.5, S. 759-765; Ya. Peng, F. Yi, G. Song, Yi. Zhang, Monatsh. Chem., 136 (2005), Nr. 10, S. 1751-1755; J. F. Dubreuil, J.P. Bazureau, Tetrahedron Lett., 41 (2000), Nr. 38, S. 7351-7356; S.-K. Fu, Sh.-T. Liu, Synth. Commun., 36 (2006), Nr. 14, S. 2059-2067; M. Yoshizawa, A. Narita, H. Ohno, Aust. J. Chem., 57 (2004), Nr.2, S. 139-144; A. Narita, W. Shibayama, H. Ohno, J. Mater. Chem., 16 (2006), Nur.15, S. 1475-1482; T. Mizumo, E. Marwanta, N. Matsumi, H. Ohno, Chem. Lett., 33 (2004), Nr. 10, S. 1360-1361; D. Zhao, Zh. Fei, T. J. Geldbach, R. Scopelliti, G. Laurenczy, P.J. Dyson, Hel. Chim. Acta., 88 (2005), Nr. 3, S. 665-675; A. Horvath, Synthesis, 1994, S. 102-106; oder für Pyrrolidinium-Kationen in L. Horner, A. Mentrup, Justus Liebigs Ann. Chem. 646 (1961), S. 49-64; Bates et al., J. Chem. Soc. 1956, S. 388-395, v.Braun Chem. Ber. 70 (1937), S. 983; Z. Dega-Szafran, R. Przybylak, J. Mol. Struct., 436 (1997), Nr. 1, S. 107-122; oder für Piperidinium-Kationen in Walther et al. , Chem. Ber., 89 (1956), S 60-65; oder für Morpholinium-Kationen in Gresham et al., J. Am. Chem. Soc., 73 (1951), S. 3168-3171; D. Le Berre, Bull. Soc. Chim. Fr., 1973, S. 2404-2407; O.A. Kazantsev, S.A. Kazakov, K.V. Shirshin, S.M. Danov, Russ. J. Org. Chem., 36 (2000), Nr. 3, S. 343-349; oder für Piperazinium-Kationen in Z. Dega-Szafran, M. Jaskolski, I. Kurzyca, P. Barczynski, M. Szafran, J. Mol. Struct, 614 (2002), Nr.1-3, S. 23-32 beschrieben sind, hergestellt werden.

Die erfindungsgemäßen Anionen sind oxidationsstabil, wie aus Tabelle 1.1 zu entnehmen ist.

**Tabelle 1.1:**

| Elektrochemische Stabilität von ionischen Flüssigkeiten mit Borat- und Phosphat-Anionen | |
|---|---|
| | E(_{Ox}), vs. Li/Li⁺ |
| 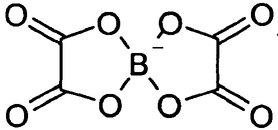 | 4,7- 4,8 V |
| Bis Oxalato Borat (BOB) | |
| 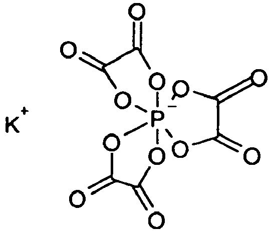 | 4,7- 4,8 V |
| Kalium Tris Oxalato Phosphat (KTOP) | |

Die Herstellung der Borat- oder Phosphat-basierten reaktiven ionischen Flüssigkeiten geschieht durch Umsetzung der entsprechenden, kationischen Onium-Chloride oder Onium-Bromide mit den entsprechenden anionischen Kalium- oder Lithium-Boraten oder -Phosphaten im wässrigen oder organischen Milieu.

Die Umsetzung der erfindungsgemäßen Kationen mit den Anionen zum Endprodukt kann bei Temperaturen von 0 bis 150°C, vorzugsweise bei 0 bis 50°C und insbesondere bei Raumtemperatur erfolgen.

Geeignete Lösungsmittel oder Lösungsmittelgemische sind Wasser bzw. VE-Wasser, Alkohole, Dioxan, Acetonitril und Aceton. Als Alkohol wird bevorzugt Methanol und iso-Propanol eingesetzt. Im Fall der Anwendung von Methyl-Phosphinaten braucht man üblicherweise kein Lösungsmittel.

Ein weiterer Gegenstand der vorliegenden Erfindung ist eine elektrochemische und/oder elektrooptische Vorrichtung enthaltend mindestens einen Elektrolyten, welcher mindestens eine ionische Flüssigkeit der allgemeinen Formel I enthält. Vorzugsweise kann die Vorrichtung eine Solarzelle, Lithium- oder Lithiumionenbatterie, ein Doppelschicht- und Superkondensator, Lithium-Kondensator, eine Licht - emittierende Vorrichtung, ein elektrochemischer Sensor und/ oder Biosensor sein.

Weiterhin-ist Gegenstand der vorliegenden Erfindung die Verwendung der genannten reaktiven ionischen Flüssigkeiten gemäß der allgemeinen Formel I als Leitsalz oder Additiv in Elektrolyten für elektrochemische oder elektrooptische Zellen.

In einer weiteren bevorzugten Ausführungsform werden die erfindungsgemäßen reaktiven ionischen Flüssigkeiten als Leitsalz oder Additiv in Elektrolyten für Batterien, sekundären Lithiumbatterien, Doppelschicht- und Superkondensatoren oder Lithium-Kondensatoren verwendet.

Weiterhin ist Gegenstand der vorliegenden Erfindung die Verwendung der erfindungsgemäßen Elektrolyten in elektrochemischen und/oder elektrooptischen Vorrichtungen. Vorzugsweise handelt es bei diesen Vorrichtungen um Lithium- oder Lithiumionen-Batterien sowie Doppelschicht- und Superkondensatoren oder Lithium-Kondensatoren.

Die folgenden Beispiele sollen die vorliegende Erfindung verdeutlichen. Sie sind jedoch keinesfalls als limitierend zu betrachten. Alle Verbindungen oder Komponenten, die in den Zubereitungen verwendet werden können, sind entweder bekannt und käuflich erhältlich oder können nach bekannten Methoden synthetisiert werden. Die in den Beispielen angegebenen Temperaturen gelten immer in °C. Es versteht sich weiterhin von selbst, dass sich sowohl in der Beschreibung als auch in den Beispielen die zugegebenen Mengen der Komponenten in den Zusammensetzungen immer zu insgesamt 100% addieren. Gegebene Prozentangaben sind immer im gegebenen Zusammenhang zu sehen. Sie beziehen sich üblicherweise aber immer auf die Masse der angegebenen Teil- oder Gesamtmenge.

### Beispiele

### Herstellung der Kationen

### Beispiel 1: Herstellung von heterozyklischen Kationen mit Allyl-Seitenketten

### Allgemeine Vorschrift:

Zu 1 mol des entsprechenden Amins werden 1,1mol eines Allylchlorids tropfenweise zugegeben. Hierbei ist darauf zu achten, dass die Temperatur zwischen 30-35°C gehalten wird. Die Reaktionsmischung wird danach 3h bis 48 h bei 40°C bis 50° C (im Falle der Bildung von festen Produkten wird die Reaktionsmischung mit Dichlormethan oder Acetonitril verdünnt) gerührt und das überschüssige Allylchlorid und Lösungsmittel danach im Vakuum (2·10⁻³ mbar) abdestilliert. Die Produktausbeute ist nahe zu quantitativ.

**Tab. 1**

| **Eingesetztes Amin** | **Eingesetztes Allylchlorid** | **Produkt** |
|---|---|---|
| 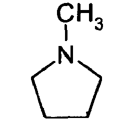 | Cl-CH₂ -CH=CH₂ | 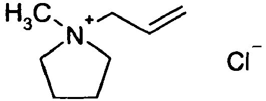 |
| 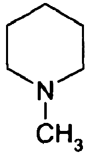 | Cl-CH₂ -CH=CH₂ | 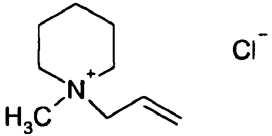 |
| 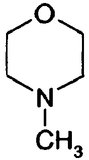 | Cl-CH₂ -CH=CH₂ | 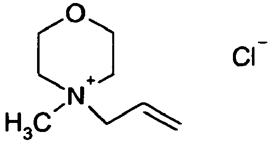 |
| 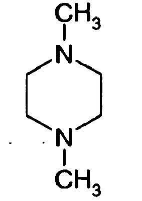 | Cl-CH₂ -CH=CH₂ | 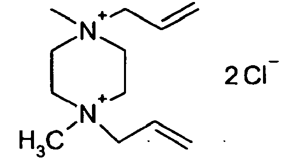 |
| 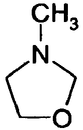 | Cl-CH₂ -CH=CH₂ | 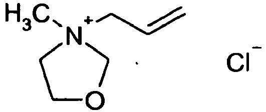 |

### Beispiel 2: Herstellung von heterozyklischen Kationen mit Carboxylat-Seitenketten

### Allgemeine Vorschrift:

In einem 2 L Mehrhalsrundkolben mit KPG-Rührer werden 1 Mol des entsprechenden Amins in 300 ml Acetonitril vorgelegt und auf 80 °C gebracht. Anschließend werden 1,1 Mol des entsprechenden Halogencarboxylats (vorzugsweise) langsam innerhalb von1,5 h hinzugetropft.

Danach wurde die Reaktionsgemisch weiterhin für 0.5 bis 48 Stunden bei dieser Temperatur reagieren lassen und in 1 L Ethylacetat eingerührt, wobei das Produkt als weißer Feststoff ausfällt. Das Produkt wird abgesaugt, mit Ethylacetat nachgewaschen und getrocknet (Rotationsverdampfer bei ca. 30°C Wasserbad). Die Ausbeute beträgt zwischen 90 und 95%

**Tab. 2**

| **Eingesetztes Amin** | **Chlor (Brom) -Alkyl-Carboxylat** | **Produkt** |
|---|---|---|
| 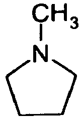 | Br-CH₂-CH₂-C(O)-O-CH₃ | 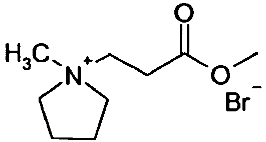 |
| 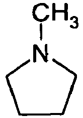 | Br -CH₂-C(O)-CH₂-C(O)-O-CH₃ | 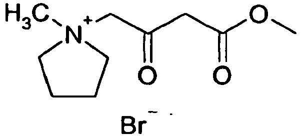 |
| 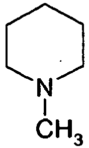 | Br -CH₂-CH₂-C(O)-O-CH₃ | 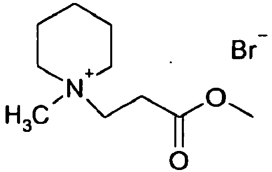 |
| 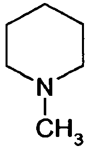 | Cl-CH₂-C(O)-CH₂-C(O)-O-CH₃ | 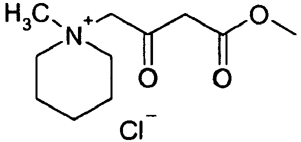 |
| 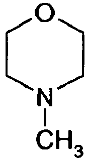 | Cl-CH₂-CH₂-C(O)-O-CH₃ | 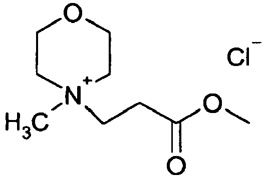 |
| 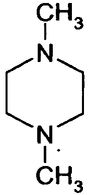 | Cl-CH₂-CH₂-C(O)-O-CH₃ | 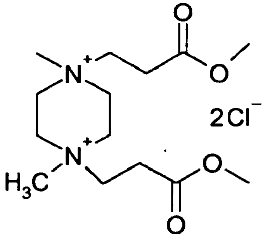 |
| 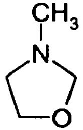 | Cl-CH₂-CH₂-C(O)-O-CH₃ | 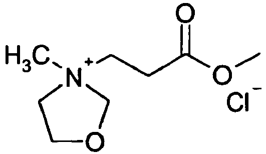 |

### Beispiel 3: Herstellung der Borat- und Phosphat-basierten ionischen Flüssigkeiten

### Allgemeine Vorschrift

In einem 3 Liter- Kolben mit Magnetfisch werden 1 mol des entsprechenden Onium-Chlorids oder Onium-Bromids (aus den vorher beschriebenen Beispielen) in 2 I Dichlormethan gelöst und anschließend langsam mit 1 Mol des entsprechenden Kalium- oder Lithiumsalzes versetzt. Nach Zugabe wird die Reaktionsmischung 5 Tage gerührt. Danach wird der Ansatz mit 500 ml VE-Wasser versetzt. Es bilden sich 2 Phasen. Die organische Phase wird abgetrennt, die wässrige Phase mit 300 ml Dichlormethan gewaschen und alle organischen Phasen vereinigt. Die organische Phase wird noch 2 mal mit ja 250 ml VE-Wasser gewaschen und danach auf 1/3 eingeengt und anschließend in 1 L n-Heptan eingerührt.

Es bilden sich zwei Phasen. Die n-Heptan-Phase mit der ionischen Flüssigkeit wird wurde über Nacht abgetrennt und am Rotationsverdampfer bei einem Wasserbad von 60 bis 70 °C eingeengt.

**Tab. 3**

| **Eingesetztes Oniumsalz** | **Eingesetztes Kalium bzw. Lithiumsalz** | **Ionische Flüssig-keit** |
|---|---|---|
| | | |
| 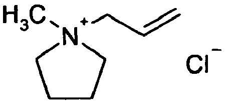 | K oder Li BOB | 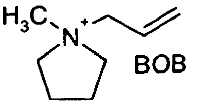 |
| 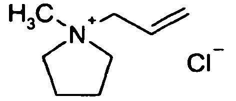 | K oder Li TOP | 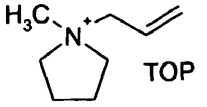 |
| 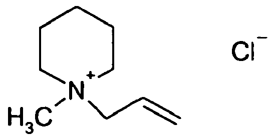 | K oder Li BOB | 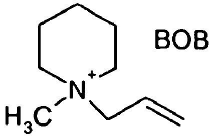 |
| 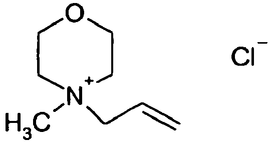 | K oder Li BOB | 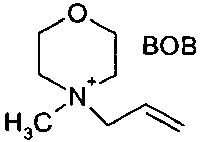 |
| 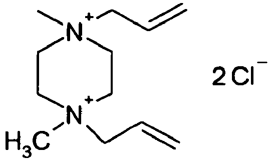 | K oder Li BOB | 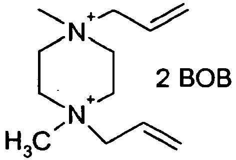 |
| 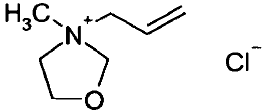 | K oder Li BOB | 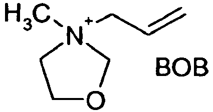 |

**Tab. 4**

| **Eingesetztes Oniumsalz** | **Eingesetztes Kalium- oder Lithium-salz** | **Ionische Flüssigkeit** |
|---|---|---|
| 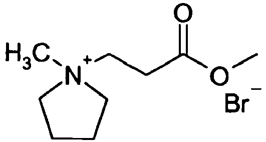 | K oder Li BOB | 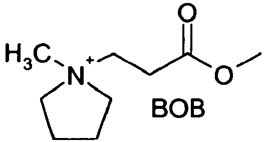 |
| 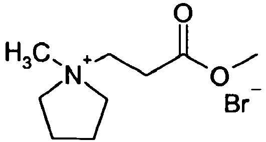 | K oder Li TOP | 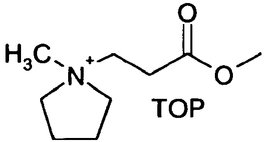 |
| | | |
| 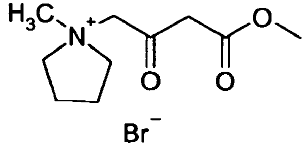 | K oder Li BOB | 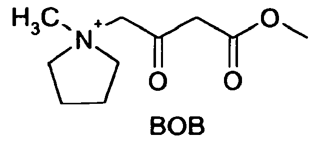 |
| 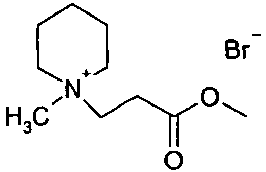 | K oder Li BOB | 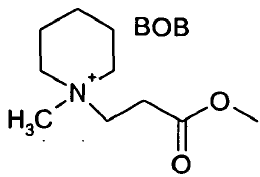 |
| | | |
| 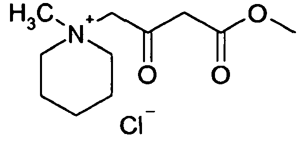 | K oder Li BOB | 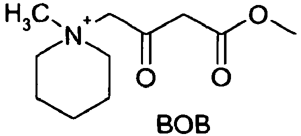 |
| 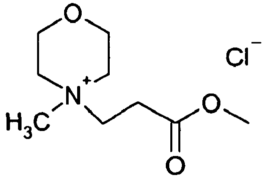 | K oder Li BOB | 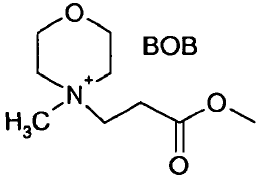 |
| | | |
| 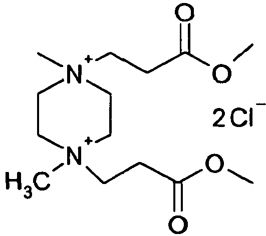 | K oder Li BOB | 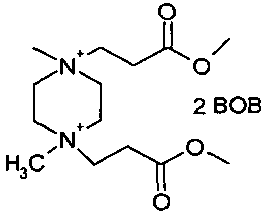 |
| 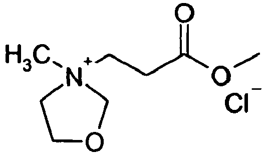 | K oder Li BOB | 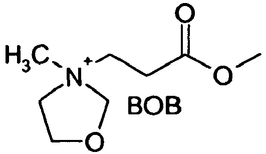 |

### Beispiel 4: Untersuchungen an Graphit

In einer Messzelle mit Graphitanode (SFG 44 mit PVDF-Binder) Lithiumgegenelektrode und Lithiumreferenzelektrode werden jeweils 5 Zyklovoltammogramme hintereinander aufgenommen. Hierzu wird ausgehend vom Ruhepotential das Potential zuerst mit einer Vorschubgeschwindigkeit von 0,1 mV/s auf 0 V gegen Li/Li⁺ erniedrigt und im weiteren Verlauf zurück auf das Ruhepotential gefahren. Als Elektrolyt wird 1M LiPF₆ in Ethylencarbonat :Diethylencarbonat (Verhältnis 3 : 7) verwendet zu dem jeweils zwischen ca. 2 % einer reaktiven ionischen Flüssigkeit ausgewählt aus Tabelle 7 gegeben wird. Die reaktive Ionische Flüssigkeit bildet bei Potentialen zwischen ca. 2 V und 0,9 V gegen Li/Li⁺ eine passivierende Deckschicht. Ab dem zweiten Zyklus wird eine 95%ige Ausbeute (± 5% Messtoleranz) der Ein- und Auslagerung von Lithiumionen in den Graphit erzielt.

Eine Kointerkalation der reaktiven Ionischen Flüssigkeit ausgewählt aus Tabelle 5 in den Graphit kann nicht beobachtet werden.

**Tab. 5**

| Ionische Flüssigkeit | Ausbeute in % | | | | |
|---|---|---|---|---|---|
| | Zyklus 1 | Zyklus 2 | Zyklus 3 | Zyklus 4 | Zyklus 5 |
| Referenz kein IL Additiv | 80 | 82 | 90 | 93 | 95 |
| 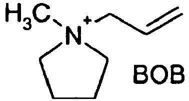 | 75 | 95 | 97 | 99 | 100 |
| 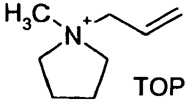 | 74 | 95 | 96 | 99 | 99 |
| 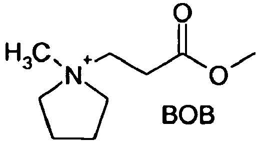 | 77 | 97 | 99 | 100 | 100 |
| 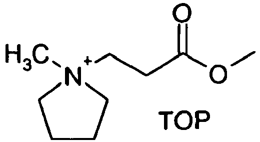 | 72 | 95 | 98 | 98 | 99 |

| | | | | | |
|---|---|---|---|---|---|
| * Referenz = LiPF₆ in EC : DEC (3:7) | | | | | |

### Beispiel 5: Untersuchungen an Graphit

In einer Messzelle mit Graphitanode (SFG 44 mit PVDF-Binder) Lithiumgegenelektrode und Lithiumreferenzelektrode werden jeweils 5 Zyklovoltammogramme hintereinander aufgenommen. Hierzu wird ausgehend vom Ruhepotential das Potential zuerst mit einer Vorschubgeschwindigkeit von 0,1 mV/s auf 0 V gegen Li/Li⁺ erniedrigt und im weiteren Verlauf zurück auf das Ruhepotential gefahren.

Als Elektrolyt wird 1 M LiPF₆ in EC:DEC (3 : 7) verwendet zu dem jeweils zwischen ca. 10% einer reaktiven Ionischen Flüssigkeit ausgewählt aus Tabelle 8 gegeben wird. Die reaktive ionische Flüssigkeit bildet bei Potentialen zwischen ca. 2 V und 0,9 V gegen Li/Li⁺ eine passivierende Deckschicht. Ab dem zweiten Zyklus wird eine über 90%ige Ausbeute der Ein- und Auslagerung von Lithiumionen in den Graphit erzielt.

Eine Kointerkalation der reaktiven ionischen Flüssigkeit aus Tabelle 6 in den Graphit kann nicht beobachtet werden.

**Tab. 6**

| Ionische Flüssigkeit (IL) | Ausbeute in % | | | | |
|---|---|---|---|---|---|
| | Zyklus 1 | Zyklus 2 | Zyklus 3 | Zyklus 4 | Zyklus 5 |
| Referenz kein IL Additiv | 80 | 82 | 90 | 93 | 95 |
| 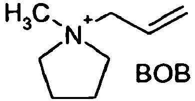 | 70 | 90 | 96 | 98 | 100 |
| | | | | | |
| 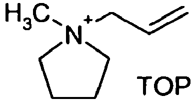 | 72 | 92 | 94 | 99 | 99 |
| 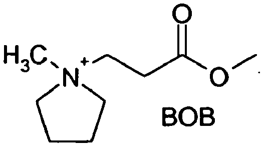 | 75 | 95 | 98 | 198 | 100 |
| 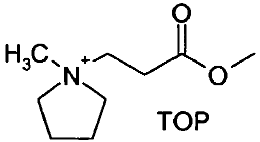 | 70 | 93 | 96 | 98 | 99 |

| | | | | | |
|---|---|---|---|---|---|
| * Referenz = LiPF₆ in EC : DEC (3:7) | | | | | |

### Beispiel 6: Untersuchungen zur Oxidationsstabilität

In einer Messzelle mit Platinarbeitselektrode, Lithiumgegenelektrode und Lithiumreferenzelektrode wurden jeweils 5 Zyklovoltammogramme hintereinander aufgenommen. Hierzu wird ausgehend vom Ruhepotential das Potential zuerst mit einer Vorschubgeschwindigkeit von 10 mV/s auf 6,0 V gegen Li/Li⁺ erhöht und im weiteren Verlauf zurück auf das Ruhepotential gefahren.

Als Elektrolyt wird 1 M LiPF₆ in EC:DEC (3 : 7) verwendet zu dem jeweils zwischen ca. 2 % einer reaktiven Ionischen Flüssigkeit ausgewählt aus Tabelle 7 gegeben wird. Das Oxidationspotential wird zu > 5V gegen Li/Li⁺ bestimmt.

In keinem der untersuchten System wird ein Signal gefunden, das unter der Oxidationsstabilität des Referenzelektrolyten liegt.

**Tab. 7**

| | Oxidationsstabilität Eₒₓ |
|---|---|
| Referenz kein IL Additiv | > 5V |
| 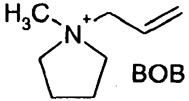 | > 5V |
| 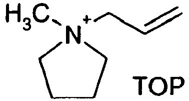 | > 5V |
| | |
| 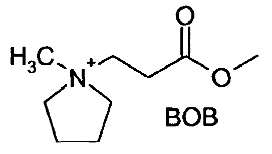 | > 5V |
| 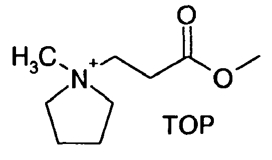 | > 5V |

| | |
|---|---|
| * Referenz = LiPF₆ in EC : DEC (3:7) | |

### Beispiel 7: Untersuchungen zur Oxidationsstabilität

In einer Messzelle mit Platinarbeitselektrode, Lithiumgegenelektrode und Lithiumreferenzelektrode werden jeweils 5 Zyklovoltammogramme hintereinander aufgenommen. Hierzu wird ausgehend vom Ruhepotential das Potential zuerst mit einer Vorschubgeschwindigkeit von 10 mV/s auf 6,0 V gegen Li/Li⁺ erhöht und im weiteren Verlauf zurück auf das Ruhepotential gefahren.

Als Elektrolyt wird 1M LiPF₆ in EC:DEC verwendet zu dem jeweils zwischen ca. 10% einer reaktiven Ionischen Flüssigkeit ausgewählt aus Tabelle 5 gegeben wird. Das Oxidationspotential wird zu > 5V gegen Li/Li⁺ bestimmt. In keinem der untersuchten Systeme wird ein Signal gefunden, das unter der Oxidationsstabilität des Referenzelektrolyten liegt.

**Tab. 8**

| Ionische Flüssigkeit | Oxidationstabilität Eox |
|---|---|
| Referenz kein IL Additiv | > 5V |
| 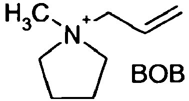 | 4,7- 4,8V |
| 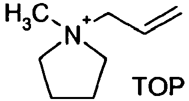 | 4,7- 4,8V |
| 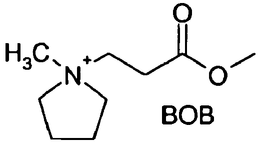 | 4,7- 4,8V |
| 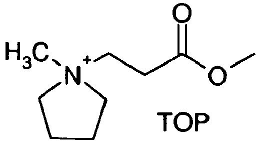 | 4,7- 4,8V |

| | |
|---|---|
| * Referenz = LiPF₆ in EC : DEC (3:7) | |

## Patentansprüche

1. Ionische Flüssigkeiten der allgemeinen Formel I
K⁺ A⁻ (I)
worin bedeuten:
K⁺ ein Kation ausgewählt aus der Gruppe der allgemeinen Formeln 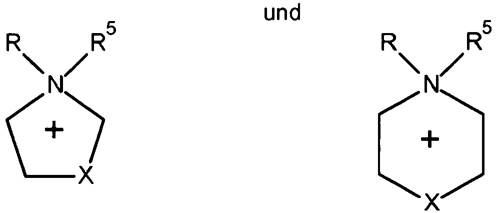
wobei
X CH₂, O, S oder NR'
R' -(CH₂)ₙ-CN, Alkyl C₁ bis C₁₆, vorzugsweise Methyl, Ethyl, Propyl, H
R H, Alkyl C₁ bis C₁₆, vorzugsweise Methyl, Ethyl, Propyl
R5 -(CH₂)ₙ-O-C(O)-R, -(CH₂)n-C(O)-OR oder -(CH₂)ₙ-HC=CH-R, wobei einzelne der CH₂-Gruppen durch S oder NR ersetzt sein können, mit n = 1 bis 8
und
A⁻ ein Anion ausgewählt aus der Gruppe 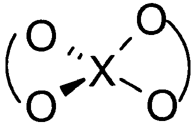 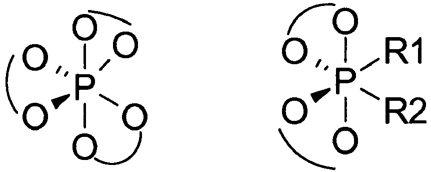
wobei 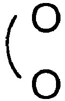
ein 1,2- oder 1,3- Diol, eine 1,2 -oder 1,3- Dicarbonsäure oder 1,2- oder 1,3- Hydroxycarbonsäure
X B
R₁ bis R₂ Halogen, insbesondere F, und/oder ein fluorierter oder nicht fluorierter Alkoxy- oder Carboxy-Rest ist.

2. Elektrolyt enthaltend mindestens ein Leitsalz, ein aprotisches Lösungsmittel oder Lösungsmittelgemisch bestehend aus organischen offenkettigen oder zyklischen Carbonaten, Carbonsäureestern, Nitrilen, Ethern oder Lactonen oder einem Gemisch daraus, mindestens eine ionische Flüssigkeit nach Anspruch 1, sowie gegebenenfalls weitere Additive.

3. Elektrolyt nach Anspruch 2, **dadurch gekennzeichnet, dass** das Leitsalz ein Lithiumleitsalz wie LiPF₆, LiN(SO₂CF₃)₂, LiN(SO₂C₂F₅)₂, LiF₃P(C₂F₅)₃, LiF₃P(C₄F₉)₃, LiB(C₂O₄)₂ oder LiF₂B(C₂O₄)₂ ist.

4. Elektrolyt nach Anspruch 2, **dadurch gekennzeichnet, dass** das Leitsalz ausgewählt wird aus folgenden Verbindungen:
N(C₂H₅)₄BF₄, N(C₂H₅)₄PF₆, N(C₂H₅)₃(CH₃)BF₄, N(C₂H₅)₃(CH₃) PF₆, N(C₂H₅)₄N(SO₂CF₃)₂, N(C₂H₅)₃(CH₃) N(SO₂CF₃)₂, N(C₂H₅)₄F₃P(C₂F₅)₃, N(C₂H₅)₃(CH₃)F₃P(C₂F₅)₃

5. Verfahren zur Herstellung von ionischen Flüssigkeiten der Formel I gemäß Anspruch 1 umfassend folgende Schritte:
• Herstellen von heterozyklischen Kationen K⁺ mit Alkenyl- oder Carboxylat-haltigen Seitenketten gemäß Anspruch 1 als Oniumchloride oder-bromide aus den entsprechenden Aminen, Halogencarboxylaten, oder Alkenylhalogeniden nach herkömmlichen nasschemischen Methoden.
• Umsetzung dieser kationischen Oniumchloride oder -bromide mit den entsprechenden
Lithium- oder Kalium-Boraten oder -Phosphaten im wässrigen und/oder alkoholischen Milieu oder organisches Lösungsmittels oder ohne Lösungsmittel.

6. Elektrochemische und/oder elektrooptische Vorrichtung enthaltend mindestens einen Elektrolyten, welcher mindestens eine ionische Flüssigkeit der allgemeinen Formel I nach Anspruch 1 enthält.

7. Elektrochemische und/oder elektrooptische Vorrichtung nach Anspruch 6, **dadurch gekennzeichnet, dass** es sich um mindestens eine Solarzelle, Lithiumionenbatterie, Lithiumbatterie, einen Doppelschicht - und Superkondensator, Lithium-Kondensator, eine Licht-emittierende Vorrichtung, einen elektrochemischen Sensor und/oder einen Biosensor handelt.

8. Verwendung von ionischen Flüssigkeiten der allgemeinen Formel I nach Anspruch 1 als Additiv in Elektrolyten für elektrochemische oder elektrooptische Zellen.

9. Verwendung von ionischen Flüssigkeiten der Formel I nach Anspruch 1 als Additiv in Elektrolyten für Batterien.

10. Verwendung von ionischen Flüssigkeiten der Formel I nach Anspruch 1 als Additiv in Elektrolyten in sekundären Lithiumbatterien.

11. Verwendung von ionischen Flüssigkeiten der Formel I nach Anspruch 1 in Doppelschicht- und Superkondensatoren oder Lithium-Kondensatoren.

12. Verwendung von ionischen Flüssigkeiten der Formel I nach Anspruch 1 als passivierende Deckschicht bildende Additive in elektrochemischen Zellen.

13. Verwendung von Elektrolyten nach einem oder mehreren der Ansprüche 2 bis 4 in elektrochemischen und/oder elektrooptischen Vorrichtungen.

14. Verwendung von Elektrolyten nach einem oder mehreren der Ansprüche 2 bis 4 in Lithium- oder Lithiumionen-Batterien.

15. Verwendung von Elektrolyten nach einem oder mehreren der Ansprüche 2 bis 4 in Doppelschicht- und Superkondensatoren oder Lithium-Kondensatoren.

## Claims

1. Ionic liquids of the general formula I
K⁺ A⁻ (I)
wherein:
K⁺ is a cation selected from the group of general formulae 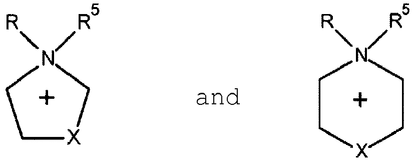 in which
X is CH₂, 0, S or NR'
R' is -(CH₂)ₙ-CN, alkyl C₁ to C₁₆, preferably methyl, ethyl, propyl, H
R is H, alkyl C₁ to C₁₆, preferably methyl, ethyl, propyl
R5 iS -(CH₂)ₙ-O-C(O)-R, -(CH₂)ₙ-C(O)-OR or -(CH₂)ₙ-HC=CH-R, where individual CH₂ groups may be replaced by S or NR,
where n = 1-8
and
A⁻ is an anion selected from the group 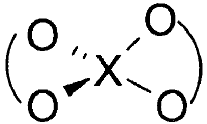 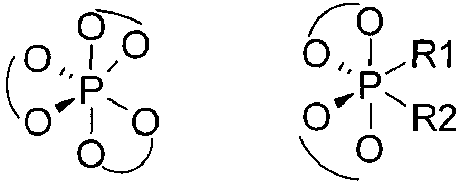 wherein 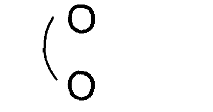
is a 1,2- or 1,3-diol, a 1,2- or 1,3-dicarboxylic acid or 1,2- or 1,3-hydroxy carboxylic acid
X is B
R₁ to R₂ are halogen, in particular F, and/or a fluorinated or non-fluorinated alkoxy or carboxy radical.

2. Electrolyte comprising at least one conducting salt, an aprotic solvent or solvent mixture consisting of organic open-chain or cyclic carbonates, carboxylic esters, nitriles, ethers or lactones or a mixture thereof, at least one ionic liquid according to claim 1, and optionally further additives.

3. Electrolyte according to claim 2, **characterized in that** the conducting salt is a lithium conducting salt such as LiPF₆, LiN (SO₂CF₃)₂, LiN (SO₂C₂F₅)₂, LiF₃P (C₂F₅)₃, LiF₃P (C₄F₉)₃, LiB(C₂O₄)₂ or LiF₂B (C₂O₄)₂.

4. Electrolyte according to claim 2, **characterized in that** the conducting salt is selected from the following compounds: N(C₂H₅)₄BF₄, N(C₂H₅)₄PF₆, N(C₂H₅)₃(CH₃)BF₄, N(C₂H₅)₃(CH₃)PF₆, N(C₂H₅)₄N(SO₂CF₃)₂, N(C₂H₅)₃(CH₃)N(SO₂CF₃)₂, N(C₂H₅)₄F₃P(C₂F₅)₃, N(C₂H₅)₃(CH₃)F₃P(C₂F₅)₃.

5. Process for the preparation of ionic liquids of the formula I according to claim 1 comprising the following steps:
• manufacturing of heterocyclic cations K⁺ with alkenyl- or carboxylate-containing side chains according to claim 1 as onium chlorides or onium bromides from the corresponding amines, halogencarboxylates or alkenyl halides by conventional wet chemical methods.
• reacting these cationic onium chlorides or onium bromides with the corresponding lithium or potassium borates or phosphates in aqueous and/or alcoholic medium or organic solvent or without solvent.

6. Electrochemical and/or electro-optical device comprising at least one electrolyte comprising at least one ionic liquid of the general formula I according to claim 1.

7. Electrochemical and/or electro-optical device according to claim 6, **characterized in that** it comprises at least one solar cell, lithium ion battery, lithium battery, double layer capacitor and super capacitor, lithium capacitor, light-emitting device, electrochemical sensor and/or biosensor.

8. The use of ionic liquids of general formula I according to claim 1 as an additive in electrolytes for electrochemical or electro-optical cells.

9. The use of ionic liquids of the formula I according to claim 1 as an additive in electrolytes for batteries.

10. The use of ionic liquids of the formula I according to claim 1 as an additive in electrolytes in secondary lithium batteries.

11. The use of ionic liquids of the formula I according to claim 1 in double layer capacitors and super capacitors or lithium capacitors.

12. The use of ionic liquids of the formula I according to claim 1 as additives forming a passivating outer layer in electrochemical cells.

13. The use of electrolytes according to one or more of claims 2 to 4 in electrochemical and/or electro-optical devices.

14. The use of electrolytes according to one or more of claims 2 to 4 in lithium or lithium ion batteries.

15. The use of electrolytes according to one or more of claims 2 to 4 in double layer capacitors and super capacitors or lithium capacitors.

## Revendications

1. Liquides ioniques de formule générale I
K⁺ A⁻ (I)
dans laquelle :
K⁺ signifie un cation choisi dans le groupe des formules générales 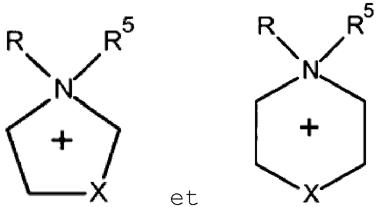 dans lesquelles
X signifie CH₂, 0, S ou NR',
R' signifie -(CH₂)ₙ-CN, alkyle en C₁ à C₁₆, de préférence méthyle, éthyle, propyle, H,
R signifie H, alkyle en C₁ à C₁₆, de préférence méthyle, éthyle, propyle,
R5 signifie -(CH₂)ₙ-O-C(O)-R, - (CH₂)ₙ-C(O)-OR ou-(CH₂)ₙ-HC=CH-R, des groupes CH₂ individuels pouvant être remplacés par S ou NR,
avec n = 1 à 8,
et
A⁻ signifie un anion choisi dans le groupe 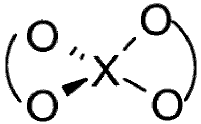 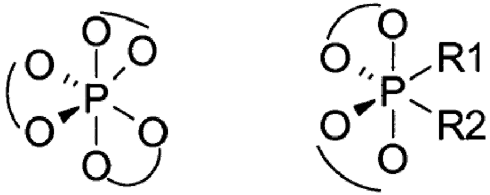 dans lesquelles 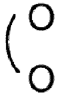
signifie un 1,2- ou 1,3-diol, un acide 1,2- ou 1,3-dicarboxylique ou un acide 1,2- ou 1,3-hydroxycarboxylique,
X signifie B,
R₁ à R₂ signifient halogène, notamment F, et/ou un radical alcoxy ou carboxy fluoré ou non fluoré.

2. Électrolyte contenant au moins un sel conducteur, un solvant aprotique ou un mélange de solvants constitué par des carbonates organiques à chaîne ouverte ou cycliques, des esters d'acides carboxyliques, des nitriles, des éthers ou des lactones ou un mélange de ceux-ci, au moins un liquide ionique selon la revendication 1 et éventuellement d'autres additifs.

3. Électrolyte selon la revendication 2, **caractérisé en ce que** le sel conducteur est un sel conducteur de lithium tel que LiPF₆, LiN (SO₂CF₃)₂, LiN (SO₂C₂F₅)₂, LiF₃P(C₂F₅)₃, LiF₃P(C₄F₉)₃, LiB(C₂O₄)₂ ou LiF₂B(C₂O₄)₂.

4. Électrolyte selon la revendication 2, **caractérisé en ce que** le sel conducteur est choisi parmi les composés suivants : N(C₂H₅)₄BF₄, N(C₂H₅)₄PF₆, N(C₂H₅)₃(CH₃)BF₄, N(C₂H₅)₃(CH₃)PF₆, N(C₂H₅)₄N(SO₂CF₃)₂, N(C₂H₅)₃(CH₃)N(SO₂CF₃)₂, N(C₂H₅)₄F₃P(C₂F₅)₃, N(C₂H₅)₃(CH₃)F₃P(C₂F₅)₃.

5. Procédé de fabrication de liquides ioniques de formule I selon la revendication 1, comprenant les étapes suivantes :
- la fabrication de cations hétérocycliques K⁺ avec des chaînes latérales contenant des groupes alcényle ou carboxylate selon la revendication 1 sous la forme de chlorures ou de bromures d'onium à partir des amines, halogénocarboxylates ou halogénures d'alcényle correspondants selon des méthodes de chimie humide habituelle,
- la mise en réaction de ces chlorures ou bromures d'onium cationiques avec les borates ou phosphates de lithium ou de potassium correspondants dans un milieu aqueux et/ou alcoolique ou un solvant organique ou sans solvant.

6. Dispositif électrochimique et/ou électro-optique contenant au moins un électrolyte, qui contient au moins un liquide ionique de formule générale I selon la revendication 1.

7. Dispositif électrochimique et/ou électro-optique selon la revendication 6, **caractérisé en ce qu'**il s'agit d'au moins une cellule solaire, une batterie au lithium-ion, une batterie au lithium, un condensateur double couche et un super-condensateur, un condensateur au lithium, un dispositif électroluminescent, un capteur électrochimique et/ou un biocapteur.

8. Utilisation de liquides ioniques de formule générale I selon la revendication 1 en tant qu'additif dans des électrolytes pour cellules électrochimiques ou électro-optiques.

9. Utilisation de liquides ioniques de formule I selon la revendication 1 en tant qu'additif dans des électrolytes pour batteries.

10. Utilisation de liquides ioniques de formule I selon la revendication 1 en tant qu'additif dans des électrolytes dans des batteries au lithium secondaires.

11. Utilisation de liquides ioniques de formule I selon la revendication 1 dans des condensateurs double couche et des super-condensateurs ou des condensateurs au lithium.

12. Utilisation de liquides ioniques de formule I selon la revendication 1 en tant qu'additifs formant une couche supérieure de passivation dans des cellules électrochimiques.

13. Utilisation d'électrolytes selon une ou plusieurs des revendications 2 à 4 dans des dispositifs électrochimiques et/ou électro-optiques.

14. Utilisation d'électrolytes selon une ou plusieurs des revendications 2 à 4 dans des batteries au lithium ou au lithium-ion.

15. Utilisation d'électrolytes selon une ou plusieurs des revendications 2 à 4 dans des condensateurs double couche et des super-condensateurs ou des condensateurs au lithium.
